(19) Europäisches Patentamt
European Patent Office
Office européen des brevets

(11) Veröffentlichungsnummer : **0 388 871 B1**

# (12) EUROPÄISCHE PATENTSCHRIFT

(45) Veröffentlichungstag der Patentschrift : **13.01.93 Patentblatt 93/02**

(51) Int. Cl.$^5$ : **A01N 43/653,** A01N 43/50

(21) Anmeldenummer : **90105196.1**

(22) Anmeldetag : **20.03.90**

## (54) Herbizide und pflanzenwachstumsregulierende Azolylmethyloxirane.

(30) Priorität : **21.03.89 DE 3909222**
**11.07.89 DE 3922770**
**22.09.89 DE 3931651**

(43) Veröffentlichungstag der Anmeldung : **26.09.90 Patentblatt 90/39**

(45) Bekanntmachung des Hinweises auf die Patenterteilung : **13.01.93 Patentblatt 93/02**

(84) Benannte Vertragsstaaten : **BE CH DE ES FR GB IT LI NL**

(56) Entgegenhaltungen :
**EP-A- 0 196 038**
**EP-A- 0 315 850**
**DE-A- 3 111 238**
**DE-A- 3 218 130**
**DE-A- 3 537 817**

(73) Patentinhaber : **BASF Aktiengesellschaft**
**Carl-Bosch-Strasse 38**
**W-6700 Ludwigshafen (DE)**

(72) Erfinder : **Seele, Rainer, Dr.**
**Leiblstrasse 3**
**W-6701 Fussgoenheim (DE)**
Erfinder : **Kober, Reiner, Dr.**
**Im Schlittweg 20**
**W-6701 Fussgoenheim (DE)**
Erfinder : **Karbach, Stefan, Dr.**
**Grundwiesenweg 44**
**W-6730 Neustadt (DE)**
Erfinder : **Sauter, Hubert, Dr.**
**Neckarpromenade 20**
**W-6800 Mannheim 1 (DE)**
Erfinder : **Wuerzer, Bruno, Dr.**
**Ruedigerstrasse 13**
**W-6701 Otterstadt (DE)**
Erfinder : **Westphalen, Karl-Otto, Dr.**
**Mausbergweg 58**
**W-6720 Speyer (DE)**
Erfinder : **Rohr, Wolfgang, Dr.**
**In der Dreispitz 13**
**W-6706 Wachenheim (DE)**
Erfinder : **Rademacher, Wilhelm, Dr.**
**Austrasse 1**
**W-6703 Limburgerhof (DE)**
Erfinder : **Jung, Johann, Prof., Dr.**
**Hardenburgstrasse 19**
**W-6703 Limburgerhof (DE)**

EP 0 388 871 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von Azolylmethyloxiranen der allgemeinen Formel I,

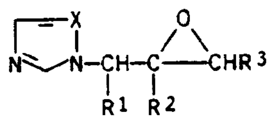

I

in der die Substituenten folgenden Bedeutung haben:

$R^1$ Wasserstoff oder ein Halogenatom,

$R^2$, $R^3$ $C_1$-$C_8$-Alkyl, welches einen der folgenden Reste tragen kann: Dioxanyl oder Phenyl, wobei der Phenylrest seinerseits ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy und/oder $C_1$-$C_4$-Halogenalkoxy,

$C_3$-$C_8$-Cycloalkyl, $C_5$-$C_8$-Cycloalkenyl, Tetrahydropyranyl oder Norbornyl, wobei diese cyclischen Reste ein bis drei der folgenden Substituenten tragen können: $C_1$-$C_4$-Alkyl und/oder Halogen,

Phenyl, Biphenyl, Naphthyl oder Pyridyl, wobei diese aromatischen Reste ein bis fünf Halogenatome und/oder ein bis drei der folgenden Substituenten tragen können: $C_1$-$C_4$-Alkyl, $C_1$-$C_4$-Halogenalkyl, $C_1$-$C_4$-Alkoxy, $C_1$-$C_4$-Halogenalkoxy, Phenoxy, Phenylsulfonyl, Amino, $C_1$-$C_4$-Alkylamino, Di-$C_1$-$C_4$-Alkylamino und/oder Nitro,

X Stickstoff oder eine Methingruppe CH

oder deren landwirtschaftlich brauchbaren Salze als Herbizide, die Verwendung von Azolylmethyloxiranen der allgemeinen Formel I, in der $R^1$ nicht Wasserstoff bedeutet als pflanzenwachstumsregulierende Mittel sowie Verfahren zur Anwendung der herbiziden bzw. pflanzenwuchsregulierenden Mittel.

Die DE-A 3 536 529, die DE-A 3 825 814 und die EP-A 94 564 beschreiben die eingangs definierten Azolylmethyloxirane I als Fungizide. Außerdem werden in der EP-A 315 850 die eingangs definierten Azolylmethyloxirane, in denen $R^1$ Wasserstoff bedeutet als Wachstumsregulatoren beschrieben.

Aufgabe der vorliegenden Erfindung war es, neue herbizide und pflanzenwachstumsregulierende Mittel zu finden.

Entsprechend dieser Aufgabe wurde gefunden, daß sich Azolylmethyloxirane sowohl zur Bekämpfung unerwünschten Pflanzenwachstums als auch zur Beeinflussung des Pflanzenwuchses eignen.

Man erhält die Azolylmethyloxirane I nach allgemein bekannten Methoden (z.B. DE-A 3 563 529 und EP-A 94 564).

Verbindungen I, in denen $R^1$ ein Halogenatom bedeutet, erhält man besonders vorteilhaft durch Umsetzung eines Epoxyaldehyds II mit einem N-Methylazolderivat III in Gegenwart eines Halogenierungsmittels (Hal).

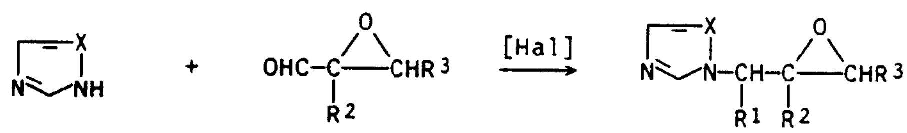


III II I

Geeignete Halogenierungsmittel sind dabei Phosphorhalogenide wie Phosphortrichlorid, Phosphortribromid, Phosphorpentachlorid, des weiteren Phosphoroxyhalogenide wie Phosphoroxytrichlorid und Thionylhalogenide wie Thionylchlorid und Thionylbromid.

Die Umsetzung wird üblicherweise in Gegenwart oder Abwesenheit eines aprotischen organischen Lösungsmittels bei Temperaturen von -30°C bis 80°C, insbesondere 0°C bis 30°C, durchgeführt.

Dabei arbeitet man bevorzugt ohne Lösungsmittel oder in aprotischen Lösungsmitteln wie Pentan, Hexan, Dekalin, Toluol, Methylenchlorid, Chloroform, Tetrachlorkohlenstoff, Dichlorethan oder entsprechenden Gemischen.

Die Verbindungen der Formel I enthalten asymmetrische C-Atome und können daher als Enantiomere und Diastereomere auftreten. Die Gemische von Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Die Racemate lassen sich bei den erfindungsgemäßen Verbindungen nach bekannten Methoden, beispielsweise durch Salzbildung mit einer optisch aktiven Säure, Trennung der diastereomeren Salze und Freisetzung der Enantiomeren mittels einer Base trennen. Als herbizide bzw. wachstumsregulierende Mittel eignen sich sowohl die einheitlichen Diastereomere bzw. Enantiomere als auch deren bei der Synthese anfallenden Gemische.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der Azolylmethyloxirane mit den Säuren.

Im Hinblick auf ihre Verwendung als Herbizide eignen sich besonders Azolylmethyloxirane I, in denen die Substituenten folgende Bedeutung haben:

$R^1$ Wasserstoff oder ein Halogenatom wie Fluor, Chlor, Brom und Jod, insbesondere Wasserstoff, Chlor und Brom,

$R^2$ eine Alkylgruppe wie Methyl, Ethyl, Propyl, 1-Methylethyl, Butyl, 1-Methylpropyl, 2-Methylpropyl und 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere 1-Methylethyl und 1,1-Dimethylethyl,

wobei diese Alkylgruppe vorzugsweise in 1-, 2- oder 3-Position durch einen der folgenden Reste substituiert sein kann: 1,3-Dioxan-2-yl, 1,3-Dioxan-4-yl, 1,3-Dioxan-5-yl und 1,3-Dioxan-2-yl, insbesondere 1,3-Dioxan-2-yl oder Phenyl, welches seinerseits ein bis fünf der unter $R^1$ genannten Halogenatome, insbesondere Phenyl,

und/oder ein bis drei der folgenden Gruppen tragen kann: Alkyl mit 1 bis 4 Kohlenstoffatomen wie vorstehend genannt, insbesondere Methyl,

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Trifluormethyl,

Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy,

und/oder Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy,

eine Cycloalkylgruppe wie Cyclopropyl, Cyclobutyl, Cyclopentyl, Cyclohexyl, Cycloheptyl, und Cyclooctyl, insbesondere Cyclohexyl,

eine Cycloalkenylgruppe wie 1-Cyclopentenyl, 2-Cyclopentenyl, 3-Cyclopentenyl, 1-Cyclohexenyl, 2-Cyclohexenyl, 3-Cyclohexenyl, 1-Cycloheptenyl, 2-Cycloheptenyl, 3-Cycloheptenyl, 4-Cycloheptenyl, 1-Cyclooctenyl, 2-Cycloctenyl, 3-Cycloctenyl und 4-Cyclooctenyl, insbesondere 3-Cyclohexenyl,

ein Tetrahydropyranylrest wie 2-Tetrahydropyranyl, 3-Tetrahydropyranyl und 4-Tetrahydropyranyl, insbesondere 4-Tetrahydropyranyl,

ein Norbornylrest wie 2-Norbornyl,

wobei die vorstehend genannten cyclischen Reste ein bis drei der vorstehend genannten Alkylgruppen, insbesondere Methyl

und/oder Halogen wie Fluor, Chlor und Brom, insbesondere Chlor und Brom tragen können;

Phenyl, Biphenyl, Naphthyl oder Pyridyl, wobei diese aromatischen Reste ein bis fünf Halogenatome wie unter $R^1$ genannt, insbesondere Chlor und Brom und/oder ein bis drei der folgenden Substituenten tragen können:

Alkyl wie vorstehend genannt, insbesondere Methyl,

Halogenalkyl wie vorstehend genannt, insbesondere Trifluormethyl und Difluormethyl,

Alkoxy wie vorstehend genannt, insbesondere Methoxy,

Halogenalkoxy wie vorstehend genannt, insbesondere Trifluormethoxy,

Phenoxy, Phenylsulfonyl, Amino, Nitro, Alkylamino wie Methylamino, Ethylamino, Propylamino, 1-Methylethylamino, Butylamino, 1-Methylpropylamino, 2-Methylpropylamino und 1,1-Dimethylethylamino, insbesondere Nitro und Amino und/oder

Dialkylamino wie Dimethylamino, Diethylamino, Dipropylamino, Di-(1-methylethyl)amino, Dibutylamino, Di-(1-methylpropyl)amino, Di-(2-methylpropyl)amino und Di-(1,1-dimethylethyl)amino insbesondere Dimethylamino,

$R^3$ im allgemeinen und im besonderen die unter $R^2$ genannten Reste und

X Stickstoff oder eine Methingruppe CH.

Im Hinblick auf ihre Verwendung als Pflanzenwachstumsregulatoren eignen sich besonders Azolylmethyloxirane I, in denen die Substituenten folgende Bedeutung haben:

$R^1$ ein Halogenatom wie insbesondere Chlor und Brom,

$R^2$ eine Alkylgruppe wie insbesondere Methyl, 1-Methylethyl und 1,1-Dimethylethyl,

wobei diese Alkylgruppe vorzugsweise in 1 oder 2-Position durch folgende Reste substituiert sein kann:

Phenyl, Biphenyl, Naphthyl oder Pyridyl, wobei diese aromatischen Reste ein bis fünf Halogenatome wie unter $R^1$ genannt, insbesondere Chlor und Brom und/oder ein bis drei der folgenden Substituenten tragen können:

Alkyl wie vorstehend genannt, insbesondere Methyl,

Halogenalkyl wie vorstehend genannt, insbesondere Trifluormethyl und Difluormethyl,

Alkoxy wie vorstehend genannt, insbesondere Methoxy,

Halogenalkoxy wie vorstehend genannt, insbesondere Trifluormethoxy,

$R^3$ insbesondere die vorstehend für $R^2$ genannten Reste und

X insbesondere Stickstoff oder eine Methingruppe CH.

Die Azolylmethyloxirane I bzw. die sie enthaltenden herbiziden und wachstumsregulierenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen, Bestreichen oder Gießen angewendet werden. Bei wachstumsregulierenden Mitteln sind auch Saatgutbehandlungen möglich. Die Anwendungformen richten sich nach den Verwendungszwecken; sie sollten in jedem Fall möglichst die feinste Verteilung der erfindungsgemäßen Wirkstoffe gewährleisten.

Die Verbindungen eignen sich allgemein zur Herstellung von direkt versprühbaren Lösungen, Emulsionen, Pasten oder Öldispersionen. Als Formulierungshilfsmittel kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Die Formulierungen enthalten zwischen 0,1 und 95 Gew.%, vorzugsweise zwischen 0,5 und 90 Gew.% Wirkstoff.

Die herbiziden Wirkstoffe können beispielsweise wie folgt formuliert werden. Die Wirkstoffe werden dabei in einer Reinheit von 95 bis 100% (NMR-Reinheit) aufbereitet.

I. Man vermischt 90 Gewichtsteile der Verbindung Nr. 109 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 117 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 115 werden in einer Mischung gelöst, die aus 40 Gewichtsteilen Cyclohexanon, 30 Gewichtsteilen Isobutanol, 20 Gewichtsteilen des Anlagerungsproduktes von 7 Mol

Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 109 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 110 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des wirkstoffs enthält.

VI. 3 Gewichtsteile des Wirkstoffs Nr. 112 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 117 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 116 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. 40 Gewichtsteile der Verbindung Nr. 109 werden mit 10 Teilen Natriumsalz eines Phenolsulfonsäureharnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

Die wachstumsregulierenden Wirkstoffe werden im allgemeinen entsprechend den herbiziden Wirkstoffen formuliert.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen bzw. die daraus hergestellten gebrauchsfertigen Zubereitungen wie Lösungen, Emulsionen, Suspensionen, Pulver, Stäube, Pasten und Granulate werden in bekannter Weise, beispielsweise im Vorauflaufverfahren oder im Nachauflaufverfahren appliziert oder als Beizmittel verwendet.

Sofern die herbiziden Wirkstoffe für gewisse Kulturpflanzen weniger verträglich sind, können Ausbringungstechniken angewandt werden, bei denen diese Mittel in der Art appliziert werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit, Zielpflanzen und Wachstumsstadium 0,001 bis 5, vorzugsweise 0,01 bis 3 kg/ha aktive Substanz (a.S.).

In Anbetracht der Vielseitigkeit der Applikationsmethoden können die erfindungsgemäßen herbiziden Mittel in einer großen Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |

| Botanischer Name | Deutscher Name |
|---|---|
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |

| Botanischer Name | Deutscher Name |
|---|---|
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Die Azolylmethyloxirane der allgemeinen Formel I, in denen $R^1$ nicht Wasserstoff bedeutet, können praktisch alle Entwicklungsstadien einer Pflanze verschiedenartig beeinflussen und werden deshalb auch als Wachstumsregulatoren eingesetzt. Die Wirkungsvielfalt der Verbindungen hängt von verschiedenen Faktoren ab. Maßgeblich sind dabei vor allem

a) Pflanzenart und -sorte,

b) Zeitpunkt der Applikation, bezüglich Entwicklungsstadium der Pflanze und Jahreszeit,

c) Applikationsort und -verfahren (Samenbeize, Bodenbehandlung oder Blattapplikation)

d) klimatische Faktoren, z.B. Temperatur, Niederschlagsmenge, außerdem auch Tageslänge und Lichtintensität

e) Bodenbeschaffenheit (einschließlich Düngung),

f) Formulierung bzw. Anwendungsform des Wirkstoffs und

g) Aufwandmenge und Wirkstoffgehalt der Mittel.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, sind nachstehend einige Beispiele aufgeführt.

A. Mit den wachstumsregulierenden Azolylmethyloxiranen I ($R^1 \neq H$) läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Bei Obstgehölzen und anderen Bäumen oder Sträuchern können dadurch kostenintensive Schnittmaßnahmen reduziert werden.

Von wirtschaftlichem Interesse ist auch die Erhöhung der Standfestigkeit von lageranfälligen Kulturen wie Getreide, Reis, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanzen ermöglicht.

Durch Anwendung der Verbindungen I ($R^1 \neq H$) kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den Verbindungen I ($R^1 \neq H$) läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den wachstumsregulierenden Mitteln auf der Basis von Azolylmethyloxiranen I ($R^1 \neq H$) lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die Azolylmethyloxirane der Formel I ($R^1 \neq H$) können Ertragssteigerungen durch Eingriffe in den pflanzlichen Stoffwechsel bzw. durch Förderung oder Hemmung des vegetativen und/oder des generativen Wachstums verursachen.

C. Mit den Azolylmethyloxiranen I ($R^1 \neq H$) lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt- und Sproßachse ist auch für ein gut kontrollierbares Entblättern von Nutzpflanzen z.B. Baumwolle wesentlich.

D. Mit den Azolylmethyloxiranen I ($R^1 \neq H$) kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Verträglichkeit der Pflanzen für die Verbindungen I ($R^1 \neq H$) kann die Aufwandmenge stark variiert werden. Bei der Saatgutbehandlung werden im allgemeinen Wirkstoffmengen von 0,001 bis 50 g je Kilogramm Saatgut, vorzugsweise 0,01 bis 1 g, benötigt. Für die Blatt- und Bodenbehandlung sind im allgemeinen Gaben von 0,01 bis 10 kg/ha, bevorzugt 0,1 bis 5 kg/ha ausreichend.

Zur Verbreiterung des Wirkungsspektrums und zur Erzielung synergistischer Effekte können die herbiziden oder wachstumsregulierenden Azolylmethyloxirane I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die Verbindungen der Formel I allein oder in Kombination mit anderen Herbiziden oder Wachstumsregulatoren auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die in den nachstehenden Synthesebeispielen wiedergegebenen Vorschriften wurden unter entsprechender Abwandlung der Ausgangsverbindungen zur Gewinnung weiterer Verbindungen der Formel I benutzt; die erhaltenen Verbindungen sind in den nachfolgenden Tabellen mit physikalischen Angaben aufgeführt.

I. Herstellung der Ausgangsstoffe

Vorschrift 1

E/Z-2-(4-Fluorphenyl)-3-(2-chlorphenyl)-propenal

Zu einer Lösung von 35 g 2-Chlorbenzaldehyd in 300 ml Methanol werden 4,2 g Natriumhydroxid in 30 ml Wasser gegeben. Das Reaktionsgemisch wird auf 10°C gekühlt und schnell 36 g 4-Fluorphenylacetaldehyd zugesetzt, wobei die Temperatur in der Lösung auf 30-40°C ansteigt. Nach 10-stündigem Rühren bei 40°C werden die ausgefallenen Kristalle aus der abgekühlten Reaktionslösung abgesaugt.

Vorschrift 2

cis-2-Formyl-2-(4-fluorphenyl)-3-(2-chlorphenyl)-oxiran

78,2 g E-2-(4-Fluorphenyl)-3-(2-chlorphenyl)-propenal werden in 300 ml Methanol gelöst und 1 ml Natronlauge (konz.) zugesetzt. Die Reaktionslösung wird bei 0°C gerührt, während 20,5 g Wasserstoffperoxyd (ca. 50 %ig) langsam zugetropft werden, wobei die Innentemperatur von 30°C nicht überschritten wird. Nach beendeter Zugabe wird sechs Stunden bei Raumtemperatur gerührt, anschließend 100 ml Wasser zugesetzt und die entstandene Emulsion mit Methyl-tert.-butylether ausgeschüttelt. Die isolierte organische Phase wird daraufhin über Natriumsulfat getrocknet und eingeengt. Man erhält 52,5 g (63 %) cis-2-Formyl-2-(4-fluorphenyl)-3-(2-chlorphenyl)-oxiran.

II. Herstellung der Endprodukte

Beispiel 1

Zu einer Lösung von 29,7 g Triazol in 150 ml Methylenchlorid wird bei 0°C unter Stickstoffatmosphäre 12,8 g Thionylchlorid zugesetzt. Nach beendeter Zugabe wird bei Raumtemperatur für 30 Minuten gerührt und anschließend 20 g cis-2-Formyl-2-(4-fluorphenyl)-3-(2-chlorphenyl)-oxiran zugegeben. Nachdem das Reaktionsgemisch 12 - 15 Stunden bei Raumtemperatur rührte, wird der Lösung 100 ml Wasser zugesetzt und die organische Phase abgetrennt. Die verbleibende wässrige Phase wird zweimal mit Methylenchlorid ausgeschüttelt und die gesammelten organischen Phasen zweimal mit gesättigter Natriumhydrogencarbonat-Lösung gewaschen. Die isolierte organische Phase wird daraufhin über Natriumsulfat getrocknet und eingeengt, wobei 23,7 g (85 %) cis-2-[1-(1,2,4-Triazol-1-yl)-1-chlor-methyl]-2-(4-fluorphenyl)-3-(2-chlorphenyl)-oxiran als 2:1-Diastereomerengemisch erhalten werden. Man erhält aus Methyl-tert.-butylether 5,8 g des vermehrt gebildeten Diastereomeren A mit dem Schmelzpunkt 152-156°C (Verbindung Nr. 106).

Beispiel 2

Zu einer Lösung von 14,9 g Triazol in 75 ml Methylenchlorid wird bei 0°C unter Stickstoffatmosphäre 11,2 g Thionylbromid zugesetzt. Nach beendeter Zugabe wird bei Raumtemperatur für 30 Minuten gerührt und anschließend 10 g cis-2-Formyl-2-(4-fluorphenyl)-3-(2-chlorphenyl)-oxiran zugegeben. Nachdem das Reaktionsgemisch 12 - 15 Stunden bei Raumtemperatur rührte, wird der Lösung 100 ml Wasser zugesetzt und die organische Phase abgetrennt. Die verbleibende wässrige Phase wird zweimal mit Methylenchlorid ausgeschüttelt und die gesammelten organischen Phasen zweimal mit gesättigter Natriumhydrogencarbonat-Lösung gewaschen. Die isolierte organische Phase wird daraufhin über Natriumsulfat getrocknet und eingeengt, wobei 10,5 g (72 %) cis-2-[1-(1,2,4-triazol-1-yl)-1-brom-methyl]-2-(4-fluorphenyl)-3-(2-chlorp henyl)-oxiran als 2:1-Diastereomerengemisch erhalten werden. Man erhält aus Methyl-tert.-butylether 3,5 g des vermehrt gebildeten Diastereomeren A mit dem Schmelzpunkt 151-155°C (Verbindung Nr. 108).

Wirkstofftabelle

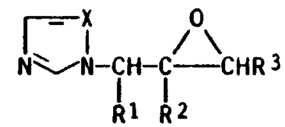

| Bsp. | $R^2$ | $R^3$ | $R^1$ | X | Schmp/IR | Isomer |
|---|---|---|---|---|---|---|
| 1 | $C_6H_5$ | 2-Cl,4-Cl-$C_6H_3$ | H | CH | 103°C | cis |
| 2 | $C_6H_5$ | 2-Cl,4-Cl-$C_6H_3$ | H | N | 159-161°C | cis |
| 3 | $C_6H_5$ | 3-$CF_3$-$C_6H_4$ | H | CH | 77-78,5°C | cis |
| 4 | $C_6H_5$ | 3-$CF_3$-$C_6H_4$ | H | N | 131-133°C | cisxHCl |
| 5 | $C_6H_5$ | $C_6H_5$ | H | CH | 108-110°C | cis |
| 6 | $C_6H_5$ | $C_6H_5$ | H | N | 116-118°C | cis |
| 7 | $C_6H_5$ | 4-Cl-$C_6H_4$ | H | CH | 105-106°C | cis |
| 8 | $C_6H_5$ | 4-Cl-$C_6H_4$ | H | N | 114-115°C | cis |
| 9 | $C_6H_5$ | 2-Cl-$C_6H_4$ | H | N | 150°C | cis |
| 10 | $C_6H_5$ | 2-F-$C_6H_4$ | H | N | 139°C | cis |
| 11 | $C_6H_5$ | 2-Br-$C_6H_4$ | H | N | 153°C | cis |
| 12 | $C_6H_5$ | 2-Cl-$C_6H_4$ | H | CH | 85- 87°C | cis/trans 15:35 |
| 13 | 4-Cl-$C_6H_4$ | $C_6H_5$ | H | CH | 138°C | cis |
| 14 | 4-Cl-$C_6H_4$ | $C_6H_5$ | H | N | 106-110°C | cis |
| 15 | 4-Cl-$C_6H_4$ | 4-$C(CH_3)_3$-$C_6H_4$ | H | CH | 160-162°C | trans |
| 16 | 4-Cl-$C_6H_4$ | 4-$C(CH_3)_3$-$C_6H_4$ | H | N | 176-177°C | cis |
| 17 | 4-Cl-$C_6H_4$ | 3-$OCH_3$-$C_6H_4$ | H | CH | 173°C | cisxHCl |
| 18 | 4-Cl-$C_6H_4$ | 3-$OCH_3$-$C_6H_4$ | H | N | 77°C | cis |
| 19 | 4-Cl-$C_6H_4$ | 4-F-$C_6H_4$ | H | N | 111-117°C | cis |

Wirkstofftabelle (Fortsetzung)

| Bsp. | $R^2$ | $R^3$ | $R^1$ | X | Schmp/IR | Isomer |
|---|---|---|---|---|---|---|
| 20 | 4-Cl-$C_6H_4$ | 4-F-$C_6H_4$ | H | CH | 103-132°C | cis |
| 21 | 4-Cl-$C_6H_4$ | 2-Cl,4-Cl-$C_6H_3$ | H | CH | 102-103°C | cis |
| 22 | 4-Cl-$C_6H_4$ | 4-Br-$C_6H_4$ | H | N | 115-119°C | cis |
| 23 | 4-Cl-$C_6H_4$ | 4-Br-$C_6H_4$ | H | CH | 114-116°C | cis |
| 24 | 4-Cl-$C_6H_4$ | 4-Br-$C_6H_4$ | H | CH | 179-181°C | trans |
| 25 | 4-Cl-$C_6H_4$ | 4-F-$C_6H_4$ | H | N | 219-223°C | trans |
| 26 | 4-Cl-$C_6H_4$ | 4-Br-$C_6H_4$ | H | N | 210-213°C | trans |
| 27 | 4-Cl-$C_6H_4$ | 4-Cl-$C_6H_4$ | H | CH | 115-120°C | cis |
| 28 | 4-Cl-$C_6H_4$ | 4-Cl-$C_6H_4$ | H | N | 105-108°C | cis |
| 29 | 4-Cl-$C_6H_4$ | 2-Cl-$C_6H_4$ | H | N | 166°C | trans |
| 30 | 4-Cl-$C_6H_4$ | 2-Cl-$C_6H_4$ | H | CH | 90- 92°C | cis/trans 70:30 |
| 31 | 4-Cl-$C_6H_4$ | 2-F-$C_6H_4$ | H | N | 138-140°C | cis |
| 32 | 4-Br-$C_6H_4$ | $C_6H_5$ | H | CH | 152-153°C | cis |
| 33 | 4-Br-$C_6H_4$ | 4-Cl-$C_6H_4$ | H | CH | 143-144°C | cis |
| 34 | 4-Br-$C_6H_4$ | 2-Cl,4-Cl-$C_6H_3$ | H | CH | 107-108°C | cis |
| 35 | 4-Br-$C_6H_4$ | $C_6H_5$ | H | N | 115-120°C | cis |
| 36 | 4-Br-$C_6H_4$ | 4-Cl-$C_6H_4$ | H | N | 115-120°C | cis |
| 37 | $C_6H_5$ | 2-Cl,5-$NO_2$-$C_6H_3$ | H | N | 130-133°C | cis |
| 38 | $C_6H_5$ | 2-$OCH_3$-$C_6H_4$ | H | CH | Harz | cis |
| 39 | $C_6H_5$ | 2-$OCH_3$-$C_6H_4$ | H | N | 115-119°C | cis |
| 40 | $C_6H_5$ | 2-$CF_3$-$C_6H_4$ | H | N | 132-134°C | cis |
| 41 | $C_6H_5$ | 2-$CF_3$-$C_6H_4$ | H | CH | 132-134°C | cis |
| 42 | 2-Cl-$C_6H_4$ | 2-Cl,4-Cl-$C_6H_3$ | H | N | Harz | cis |

Wirkstofftabelle (Fortsetzung)

| Bsp. | $R^2$ | $R^3$ | $R^1$ | X | Schmp/IR | Isomer |
|---|---|---|---|---|---|---|
| 43 | 2-Cl-$C_6H_4$ | 2-Cl,4-Cl-$C_6H_3$ | H | N | Harz | trans |
| 44 | 2-Cl-$C_6H_4$ | 2-F-$C_6H_4$ | H | N | Harz | cis/trans 3:1 |
| 45 | 2-Cl-$C_6H_4$ | 4-Cl-$C_6H_4$ | H | N | 138-147°C | cis |
| 46 | 2-Cl-$C_6H_4$ | 4-Cl-$C_6H_4$ | H | CH | 136-140°C | cis |
| 47 | 4-Cl-$C_6H_4$ | 3-Cl-$C_6H_4$ | H | CH | Harz | cis |
| 48 | 4-Cl-$C_6H_4$ | 3-Cl-$C_6H_4$ | H | N | Harz | cis |
| 49 | 2-Cl,4-Cl-$C_6H_3$ | 4-Cl-$C_6H_4$ | H | CH | 135°C | cis |
| 50 | 2-Cl,4-Cl-$C_6H_3$ | 4-$C(CH_3)_3$-$C_6H_4$ | H | CH | 176-177°C | cis |
| 51 | 2-Cl,4-Cl-$C_6H_3$ | 4-Br-$C_6H_4$ | H | CH | 135-139°C | cis |
| 52 | 2-Cl,4-Cl-$C_6H_3$ | 4-Br-$C_6H_4$ | H | N | 108-110°C | cis |
| 53 | 2-Cl,4-Cl-$C_6H_3$ | $C_6H_5$ | H | CH | Harz | cis |
| 54 | 2-Cl,4-Cl-$C_6H_3$ | $C_6H_5$ | H | CH | 118-121°C | trans |
| 55 | 2-Cl,4-Cl-$C_6H_3$ | $C_6H_5$ | H | N | Harz | cis |
| 56 | 2-Cl,4-Cl-$C_6H_3$ | $C_6H_5$ | H | N | Harz | trans |
| 57 | 2-Cl,4-Cl-$C_6H_3$ | 4-$C(CH_3)_3$-$C_6H_4$ | H | CH | 142-143°C | trans |
| 58 | 2-Cl,4-Cl-$C_6H_3$ | 2-F-$C_6H_4$ | H | N | 117°C | cis |
| 59 | 2-Cl,4-Cl-$C_6H_3$ | 2-Cl,4-Cl-$C_6H_3$ | H | N | Harz | cis |
| 60 | 2-Cl,4-Cl-$C_6H_3$ | 2-Cl,4-Cl-$C_6H_3$ | H | N | Harz | trans |
| 61 | 4-Cl-$C_6H_4$ | 3-$CF_3$-$C_6H_4$ | H | CH | 101-104°C | cis |
| 62 | 4-Cl-$C_6H_4$ | 3-$CF_3$-$C_6H_4$ | H | N | 107-109°C | cis |
| 63 | 3,4-$Cl_2$-$C_6H_3$ | 4-Br-$C_6H_4$ | H | N | 130-134°C | cis |
| 64 | 3-Br,4-F-$C_6H_3$ | 2-Cl-$C_6H_4$ | H | N | 129-130°C | cis |
| 65 | 4-$C(CH_3)_3$-$C_6H_4$ | 4-Cl-$C_6H_4$ | H | CH | 100-152°C | cis |

Wirkstofftabelle (Fortsetzung)

| Bsp. | $R^2$ | $R^3$ | $R^1$ | X | Schmp/IR | Isomer |
|---|---|---|---|---|---|---|
| 66 | $4\text{-}C(CH_3)_3\text{-}C_6H_4$ | $4\text{-}Cl\text{-}C_6H_4$ | H | N | 105-107°C | cis |
| 67 | $4\text{-}C(CH_3)_3\text{-}C_6H_4$ | $2\text{-}Cl,4\text{-}Cl\text{-}C_6H_3$ | H | CH | 101-113°C | cis |
| 68 | $4\text{-}C(CH_3)_3\text{-}C_6H_4$ | $2\text{-}Cl,4\text{-}Cl\text{-}C_6H_3$ | H | N | 108-111°C | cis |
| 69 | 4-Biphenyl | $4\text{-}Cl\text{-}C_6H_4$ | H | N | 163-165°C | cis |
| 70 | 4-Biphenyl | $2\text{-}Cl\text{-}C_6H_4$ | H | N | 191°C | cis |
| 71 | $4\text{-}CH_3\text{-}C_6H_4$ | $2\text{-}Cl\text{-}C_6H_4$ | H | N | 140°C | cis |
| 72 | $4\text{-}CH_3\text{-}C_6H_4$ | $2\text{-}F\text{-}C_6H_4$ | H | N | 131°C | cis |
| 73 | $4\text{-}OCH_3\text{-}C_6H_4$ | $2\text{-}Cl\text{-}C_6H_4$ | H | N | 135-137°C | cis |
| 74 | 2-Naphthyl | $2\text{-}Cl,4\text{-}Cl\text{-}C_6H_3$ | H | CH | 135°C | cis |
| 75 | 2-Naphthyl | $2\text{-}Cl,4\text{-}Cl\text{-}C_6H_3$ | H | N | 151°C | cis |
| 76 | 2-Naphthyl | $4\text{-}Cl\text{-}C_6H_4$ | H | N | 105-108°C | cis |
| 77 | 2-Naphthyl | $4\text{-}Cl\text{-}C_6H_4$ | H | CH | 140°C | cis |
| 78 | $4\text{-}(SO_2C_6H_5)\text{-}C_6H_4$ | $4\text{-}Cl\text{-}C_6H_4$ | H | CH | 193-195°C | cis |
| 79 | $4\text{-}(SO_2C_6H_5)\text{-}C_6H_4$ | $4\text{-}Cl\text{-}C_6H_4$ | H | CH | 204-205°C | trans |
| 80 | $4\text{-}(SO_2C_6H_5)\text{-}C_6H_4$ | $4\text{-}Cl\text{-}C_6H_4$ | H | N | 132-135°C | cis |
| 81 | $4\text{-}(SO_2C_6H_5)\text{-}C_6H_4$ | $4\text{-}Cl\text{-}C_6H_4$ | H | N | 175-177°C | trans |
| 82 | $4\text{-}F\text{-}C_6H_4$ | $2\text{-}Cl\text{-}C_6H_4$ | H | CH | 119-122°C | cis |
| 83 | $4\text{-}F\text{-}C_6H_4$ | $2\text{-}CF_3\text{-}C_6H_4$ | H | CH | 127-135°C | cis |
| 84 | $4\text{-}F\text{-}C_6H_4$ | $3\text{-}Cl\text{-}C_6H_4$ | H | CH | Harz | cis |
| 85 | $4\text{-}F\text{-}C_6H_4$ | $2\text{-}Cl,4\text{-}F\text{-}C_6H_3$ | H | CH | 135°C | cis |
| 86 | $4\text{-}F\text{-}C_6H_4$ | $4\text{-}OCF_3\text{-}C_6H_4$ | H | CH | Harz | cis |
| 87 | $4\text{-}F\text{-}C_6H_4$ | $2,6\text{-}F_2\text{-}C_6H_3$ | H | CH | 182-188°C | cis |
| 88 | $4\text{-}F\text{-}C_6H_4$ | Cyclohexyl | H | CH | 2829, 2853, 510, 1450, 1227, 838 $cm^{-1}$ | cis |

Wirkstofftabelle (Fortsetzung)

| Bsp. | $R^2$ | $R^3$ | $R^1$ | X | Schmp/IR | Isomer |
|---|---|---|---|---|---|---|
| 89 | $4-F-C_6H_4$ | $4-Cl-C_6H_4$ | H | N | 116-119°C | cis |
| 90 | $4-F-C_6H_4$ | $4-NO_2-C_6H_4$ | H | N | 110-112°C | cis |
| 91 | $4-F-C_6H_4$ | $3-NO_2-C_6H_4$ | H | N | 118-128°C | cis |
| 92 | $4-F-C_6H_4$ | $2-Cl,5-NO_2-C_6H_3$ | H | N | 133-139°C | cis |
| 93 | $4-F-C_6H_4$ | $4-NH_2-C_6H_4$ | H | N | Harz | cis |
| 94 | $4-F-C_6H_4$ | $2-Cl,5-OCH_3-C_6H_3$ | H | N | 110-112°C | cis |
| 95 | $4-F-C_6H_4$ | 2-Naphthyl | H | N | 102-104°C | cis |
| 96 | $4-F-C_6H_4$ | $2-F,6-F-C_6H_3$ | H | CH | 182-188°C | cis |
| 97 | $4-F-C_6H_4$ | $2-F,6-F-C_6H_3$ | H | N | 128-131°C | cis |
| 98 | $4-F-C_6H_4$ | $2-Cl,4-OCH_3-C_6H_3$ | H | N | 86- 90°C | cis |
| 99 | $4-F-C_6H_4$ | $3-Cl,4-F-C_6H_3$ | H | N | Harz | cis |
| 100 | $4-F-C_6H_4$ | $2-F,3-Cl-C_6H_3$ | H | N | Harz | cis |
| 101 | $4-F-C_6H_4$ | $4-OCF_3-C_6H_4$ | H | N | Harz | cis |
| 102 | $4-F-C_6H_4$ | $2-OCH_3-C_6H_4$ | H | N | 102-106°C | cis |
| 103 | $4-F-C_6H_4$ | $2-Br-C_6H_4$ | H | N | 108-113°C | cis |
| 104 | $4-F-C_6H_4$ | $2-Br-C_6H_4$ | H | CH | 122-124°C | cis |
| 105 | $4-F-C_6H_4$ | $2-OCH_3-C_6H_4$ | H | CH | Harz | cis |
| 106 | $4-F-C_6H_4$ | $2-Cl-C_6H_4$ | Cl | N | 152-156°C | Enantiomerengemisch |
| 107 | $4-F-C_6H_4$ | $2-Cl-C_6H_4$ | Cl | CH | 144-152°C | $D_1:D_2=1:1$ |
| 108 | $4-F-C_6H_4$ | $2-Cl-C_6H_4$ | Br | N | 151-155°C | Enantiomerengemisch |
| 109 | $4-F-C_6H_4$ | $2-CF_3-C_6H_4$ | Cl | N | 126-127°C | Enantiomerengemisch |
| 110 | $4-F-C_6H_4$ | $2-F,4-F-C_6H_3$ | Cl | N | Harz | $D_1:D_2=1:1$ |
| 111 | $4-F-C_6H_4$ | $2-F,4-F-C_6H_3$ | Cl | CH | 118-123°C | $D_1:D_2=1:1$ |

Wirkstofftabelle (Fortsetzung)

| Bsp. | $R^2$ | $R^3$ | $R^1$ | X | Schmp/IR | Isomer |
|---|---|---|---|---|---|---|
| 112 | 4-F-$C_6H_4$ | 3-F-$C_6H_4$ | Cl | N | Harz | $D_1$:$D_2$=2:1 |
| 113 | 4-F-$C_6H_4$ | 4-$CF_3$-$C_6H_4$ | Cl | CH | 90- 94°C | $D_1$:$D_2$=2:1 |
| 114 | 4-F-$C_6H_4$ | 2-Br-$C_6H_4$ | Cl | N | 151-158°C | Enantiomerengemisch |
| 115 | 4-F-$C_6H_4$ | 2-Br-$C_6H_4$ | Cl | CH | 159-164°C | Enantiomerengemisch |
| 116 | 4-F-$C_6H_4$ | $C_6H_5$ | Cl | N | 134-138°C | $D_1$:$D_2$=12:1 |
| 117 | 4-F-$C_6H_4$ | $C_6H_5$ | Cl | CH | Harz | $D_1$:$D_2$=1:1 |
| 118 | 4-F-$C_6H_4$ | Norbornyl | H | N | 212-215°C | cis/trans=1:1 |
| 119 | 4-F-$C_6H_4$ | 3-Cyclohexenyl | H | N | 1510, 1273, 1139, 840 $cm^{-1}$ | cis/trans=60:40 |
| 120 | 4-F-$C_6H_4$ | 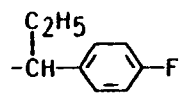 | H | N | 123-127°C | $D_1$:$D_2$=1:1 |
| 121 | 4-F-$C_6H_4$ | 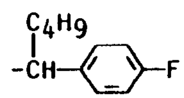 | H | N | 2958, 1605 1509, 1225, 837 $cm^{-1}$ | $D_1$:$D_2$=1:1 |
| 122 | 4-F-$C_6H_4$ | 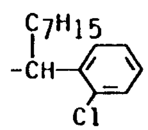 | H | N | 2954, 2928 507, 1273, 1140, 755 $cm^{-1}$ | $D_1$:$D_2$=1:1 |
| 123 | $CH_3$ | 2-Cl-$C_6H_4$ | H | N | 91°C | cis |
| 124 | tert.-$C_4H_9$ | 4-Cl-$C_6H_4$ | H | N | Harz | cis |
| 125 | tert.-$C_4H_9$ | 4-Cl-$C_6H_4$ | H | CH | Harz | cis |

Wirkstofftabelle (Fortsetzung)

| Bsp. | R2 | R3 | R1 | X | Schmp/IR | Isomer |
|---|---|---|---|---|---|---|
| 126 | 4-F-$C_6H_4$ | Cyclopropyl | H | N | 105-108°C | cis/trans 35:65 |
| 127 | 4-F-$C_6H_4$ | Cyclopentyl | H | N | 50- 56°C | cis/trans 60:40 |
| 128 | 4-F-$C_6H_4$ | Cyclohexyl | H | N | Harz | cis |
| 129 | 4-tetrahydropyranyl | 2-Cl,4-Cl-$C_6H_3$ | H | N | 155-158°C | cis |
| 130 | 4-tetrahydropyranyl | 2-Cl-$C_6H_4$ | H | N | 101-103°C | trans |
| 131 | 4-tetrahydropyranyl | 4-Cl-$C_6H_4$ | H | N | 157-163°C | trans |
| 132 | 4-tetrahydropyranyl | 2-F-$C_6H_4$ | H | N | 131-134°C | trans |
| 133 | Cyclohexyl | 2-Cl-$C_6H_4$ | H | N | 106-108°C | cis |
| 134 | Cyclohexyl | 4-Cl-$C_6H_4$ | H | N | Harz | cis/trans 50:50 |
| 135 | Cyclohexyl | 2-Cl,4-Cl-$C_6H_3$ | H | N | 109-111°C | cis |
| 136 | Cyclohexyl | 2-F-$C_6H_4$ | H | N | 90- 92°C | cis |
| 137 | Cyclohexyl | 4-F-$C_6H_4$ | H | N | 2928,2854 1511,1225,1020 $cm^{-1}$ | cis |
| 138 | Cyclohexyl | 2-Cl,4-Cl-$C_6H_3$ | H | CH | 72- 74°C | cis |
| 139 | Cyclohexyl | 4-F-$C_6H_4$ | H | CH | Harz | cis/trans 50:50 |
| 140 | $CH_2C(CH_3)_3$ | 4-Cl-$C_6H_4$ | H | N | 59- 67°C | cis/trans 50:50 |
| 141 | $CH_2C(CH_3)_3$ | Cyclohexyl | H | N | 2929,1506, 1274,1139,1016 $cm^{-1}$ | cis/trans 70:30 |
| 142 | $CH_2C(CH_3)_3$ | 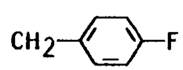 | H | N | 70- 77°C | cis/trans 50:50 |
| 143 | 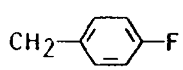 | tert.-$C_4H_9$ | H | N | 49- 67°C | cis/trans 50:50 |

Wirkstofftabelle (Fortsetzung)

| Bsp. | $R^2$ | $R^3$ | $R^1$ | X | Schmp/IR | Isomer |
|---|---|---|---|---|---|---|
| 144 | $CH_2$– 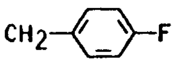 | 2-Cl-$C_6H_4$ | H | N | Harz | cis/trans 50:50 |
| 145 | $CH_2$– 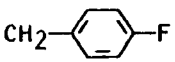 | $CH_2C(CH_3)_3$ | H | N | 75- 77°C | cis/trans 50:50 |
| 146 | $CH_2$– 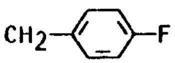 | $CH_2CH_2$– 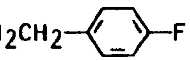 | H | N | Harz | cis/trans 50:50 |
| 147 | $CH_2$– 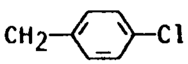 | $CH_2CH_2C(CH_3)_3$ | H | N | Harz | cis/trans 60:40 |
| 148 | $CH_2$– 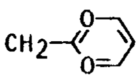 | 2-Cl-$C_6H_4$ | H | N | 96-111°C | cis |
| 149 | $C_6H_5$ | 4-F-$C_6H_4$ | H | N | 64- 73°C | cis |
| 150 | 4-F-$C_6H_4$ | 3-Tetrahydropyranyl | H | N | Harz | cis |
| 151 | $CH_3$ | 4-$C(CH_3)_3$-$C_6H_4$ | H | N | 118-121°C | cis |
| 152 | 4-$C(CH_3)_3$-$C_6H_4$ | 2-Cl-$C_6H_4$ | H | N | 122°C | cis |
| 153 | 4-F-$C_6H_4$ | 2-Cl,4-F-$C_6H_3$ | H | N | 139°C | cis |
| 154 | 4-F-$C_6H_4$ | 2-Cl,4-Cl-$C_6H_3$ | H | N | 127°C | cis |
| 155 | 4-Cl-$C_6H_4$ | 2-Cl,4-F-$C_6H_3$ | H | N | 143-145°C | cis |
| 156 | $C_6H_5$ | 2-Cl,4-F-$C_6H_3$ | H | N | 123°C | cis |
| 157 | $C_6H_5$ | 3-Pyridyl | H | N | 75°C | cis |
| 158 | $C_6H_5$ | 4-$CF_3$-$C_6H_4$ | H | N | 132°C | cis |

Wirkstofftabelle (Fortsetzung)

| Bsp. | $R^2$ | $R^3$ | $R^1$ | X | Schmp/IR | Isomer |
|---|---|---|---|---|---|---|
| 159 | 2-F-$C_6H_4$ | 2-Cl-$C_6H_4$ | H | N | 145-147°C | cis |
| 160 | $C_6H_5$ | 2-Cl,6-F-$C_6H_3$ | H | N | 158°C | cis |
| 161 | 2-F-$C_6H_4$ | 2-F-$C_6H_4$ | H | N | 128°C | cis |
| 162 | 3-Br,4-F-$C_6H_3$ | 2-F-$C_6H_4$ | H | N | 106°C | cis |
| 163 | 4-F-$C_6H_4$ | 2-$CH_3$-$C_6H_4$ | H | N | 99-102°C | cis |
| 164 | 4-F-$C_6H_4$ | 2-$CH_3$-$C_6H_4$ | H | CH | Harz | cis |
| 165 | $C(CH_3)_3$ | 2-Cl,4-$Cl_6H_3$ | H | N | 81- 89°C | cis |
| 166 | 4-F-$C_6H_4$ | 2-$OCH_3$-$C_6H_4$ | Cl | N | 118-124°C | Gemisch |
| 167 | 4-F-$C_6H_4$ | 2-$CHF_2$-$C_6H_4$ | H | N | 67- 73°C | cis |
| 168 | $C(CH_3)_3$ | 2-Cl,4-Cl-$C_6H_3$ | H | CH | Harz | cis |
| 169 | 4-F-$C_6H_4$ | 2-$CHF_2$-$C_6H_4$ | H | CH | Harz | cis |
| 170 | $C_6H_5$ | 2-Cl-$C_6H_4$ | Cl | N | 136-138°C | Gemisch |
| 171 | $C_6H_5$ | 4-Cl-$C_6H_4$ | Cl | CH | 167-172°C | Gemisch |
| 172 | $C_6H_5$ | 2-Cl,4-Cl-$C_6H_3$ | Cl | CH | 176-180°C | Gemisch |
| 173 | 4-F-$C_6H_4$ | 2-CHClF-$CF_2$-$C_6H_4$ | X | N | 88- 90°C | cis |
| 174 | 4-Cl-$C_6H_4$ | 2-Br-$C_6H_4$ | H | N | 144-149°C | cis |
| 175 | 4-Cl-$C_6H_4$ | 2-$CH_3$-$C_6H_4$ | H | CH | Harz | cis |
| 176 | 4-Cl-$C_6H_4$ | 2-$CH_3$-$C_6H_4$ | H | N | 105-108°C | cis |
| 177 | $C_6H_5$ | 2-Br-$C_6H_4$ | H | CH | 113-114°C | cis |
| 178 | 4-Cl-$C_6H_4$ | 2-$CF_3$-$C_6H_4$ | H | N | 117-119°C | cis |
| 179 | 4-Cl-$C_6H_4$ | 2-Br-$C_6H_4$ | H | CH | 142-145°C | cis |

Wirkstofftabelle (Fortsetzung)

| Bsp. | $R^2$ | $R^3$ | $R^1$ | X | Schmp/IR | Isomer |
|---|---|---|---|---|---|---|
| 180 | $C_6H_5$ | 2-Br-$C_6H_4$ | H | N | 158-160°C | cis |
| 181 | $C_6H_5$ | 2-Br-$C_6H_4$ | Cl | N | Harz | $D_1:D_2=1:1$ |
| 182 | $C_6H_5$ | 2-Br-$C_6H_4$ | H | CH | Harz | $D_1:D_2=1:1$ |
| 183 | 2-Cl-$C_6H_4$ | 2-$CF_3$-$C_6H_4$ | H | N | Harz | cis |
| 184 | 4-Cl-$C_6H_4$ | 2-$OCH_3$-$C_6H_4$ | H | N | 100-104°C | cis |
| 185 | 2-Cl-$C_6H_4$ | 2-$CF_3$-$C_6H_4$ | H | CH | Harz | cis/trans 60:40 |
| 186 | 4-Cl-$C_6H_4$ | 2-$OCH_3$-$C_6H_4$ | H | CH | Harz | cis |
| 187 | 4-F-$C_6H_4$ | 2,4-$F_2$-$C_6H_3$ | H | N | Harz | cis |
| 188 | 4-F-$C_6H_4$ | 2-F-$C_6H_4$ | Cl | CH | Harz | $D_1:D_2=3:1$ |
| 189 | 4-F-$C_6H_4$ | 2-F-$C_6H_4$ | Cl | N | Harz | $D_1:D_2=2:1$ |
| 190 | 4-F-$C_6H_4$ | 4-$C_6H_5$-O-$C_6H_4$ | H | N | Harz | cis |
| 191 | 4-F-$C_6H_4$ | 4-$C_6H_5$-O-$C_6H_4$ | H | CH | Harz | cis |
| 192 | 4-F-$C_6H_4$ | 4-$CF_3$-$C_6H_4$ | H | CH | Harz | cis |
| 193 | $C_6H_5$ | 4-Biphenyl | H | N | Harz | cis |
| 194 | 4-F-$C_6H_4$ | 1-Naphthyl | H | N | 160-163°C | cis |
| 195 | $C_6H_5$ | 4-Biphenyl | H | CH | Harz | cis |
| 196 | $C_6H_5$ | 2,4-$(CH_3)_2$-$C_6H_3$ | H | N | 134-136°C | cis |
| 197 | 4-F-$C_6H_4$ | 1-Naphthyl | H | CH | Harz | cis |
| 198 | $C_6H_5$ | 3-$C_6H_5$-O-$C_6H_4$ | H | N | Harz | cis |
| 199 | $C_6H_5$ | 3-$C_6H_5$-O-$C_6H_4$ | H | CH | Harz | cis |

Wirkstofftabelle (Fortsetzung)

| Bsp. | $R^2$ | $R^3$ | $R^1$ | X | Schmp/IR | Isomer |
|---|---|---|---|---|---|---|
| 200 | $4-CH_3-C_6H_4$ | $C_6H_5$ | H | N | 144-146°C | cis |
| 201 | $C_6H_5$ | $2-CH_3-C_6H_4$ | H | N | Harz | cis |
| 202 | $4-F-C_6H_4$ | $2,4-(CH_3)_2-C_6H_3$ | H | N | 83- 85°C | cis |
| 203 | $4-F-C_6H_4$ | $2,4-(CH_3)_2-C_6H_3$ | H | CH | Harz | cis |
| 204 | $C_6H_5$ | $2-CH_3-C_6H_4$ | H | CH | Harz | cis |
| 205 | $C_6H_5$ | $3-CH_3-C_6H_4$ | H | N | Harz | cis |
| 206 | $C_6H_5$ | $2-C_2H_5-C_6H_4$ | H | N | Harz | cis |
| 207 | $C_6H_5$ | $2-C_2H_5-C_6H_4$ | H | CH | Harz | cis |
| 208 | $C_6H_5$ | $3-CH_3-C_6H_4$ | H | CH | Harz | cis |
| 209 | $C_6H_5$ | $4-CH_3-C_6H_4$ | H | CH | Harz | cis |
| 210 | $C_6H_5$ | $4-CH_3-C_6H_4$ | H | N | 106-108°C | cis |
| 211 | $C_6H_5$ | $4-CO_2CH_3-C_6H_4$ | H | N | 150-152°C | cis |
| 212 | $4-F-C_6H_4$ | 3-Thienyl | H | N | 78- 80°C | cis |
| 213 | $C_6H_5$ | $4-CO_2CH_3-C_6H_4$ | H | CH | 112-114°C | cis |
| 214 | $4-F-C_6H_4$ | 3-Thienyl | H | CH | Harz | cis |
| 215 | $C_6H_5$ | $2,4-Cl_2-C_6H_3$ | H | CH | 50- 52°C | cis |
| 216 | $C_6H_5$ | $3,4-Cl_2-C_6H_3$ | H | CH | Harz | cis |

Anwendungsbeispiele

Die herbizide Wirkung der Azolylmethyloxirane der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 2,0 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürz. | Lateinischer Name | Deutscher Name | Englischer Name |
|---|---|---|---|
| CHEAL | Chenopodium album | Weißer Gänsefuß | lambsquarters (goosefoot) |
| GALAP | Galium aparine | Klettenlabkraut | catchweed bedstraw |
| SOLNI | Solanum nigrum | Schwarzer Nachtschatten | black nightshade |

Mit 2,0 kg/ha a.S. im Nachauflaufverfahren eingesetzt, ließen sich mit dem Beispiel 176 breitblättrige unerwünschte Pflanzen sehr gut bekämpfen.

Zur Bestimmung der wachstumsregulierenden Eigenschaften wurden die Wirkstoffe im Nachauflaufverfahren in wäßriger Aufbereitung appliziert. Die Anzucht der Pflanzen erfolgte dabei unter den vorstehend beschriebenen Gewächshausbedingungen auf ausreichend mit Nährstoffen versorgtem Kultursubstrat in Kunststoffgefäßen (ca. 12,5 cm Durchmesser, Volumen ca. 500 ml). Die beobachtete wachstumsregulierende Wirkung wurde bei Versuchsende nach ca. 14 Tagen durch Wuchshöhenmessung belegt. Die so gewonnenen Meßwerte wurden zur Wuchshöhe der unbehandelten Pflanzen in Relation gesetzt.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die wachstumsregulierende Wirkung der Azolylmethyloxirane I ($R^1 \neq H$) ist den folgenden Tabellen zu entnehmen. Als bekannter Wachstumsregulator wurde Chlormequatchlorid (CCC) parallel zu den Azolylmethyloxiranen getestet.

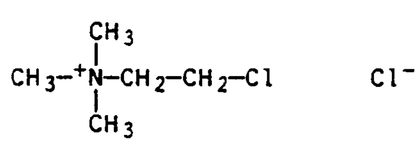

Chlormequatchlorid (CCC)

Tabelle 1

Beeinflussung des Längenwachstums von Sommerraps "Petranova" bei Nachauflauf-Blattbehandlung

| Beispiel Nr. | mg a.S./Gefäß | relative Wuchshöhen* in % |
|---|---|---|
| 109 | 6 | 78,3 |
| 110 | 6 | 80,4 |
| 112 | 6 | 74,4 |
| 113 | 6 | 88,4 |
| 115 | 6 | 79,0 |
| 116 | 6 | 72,9 |
| 117 | 6 | 85,1 |
| CCC | 6 | 93,2 |

* bezogen auf unbehandelte Pflanzen

Tabelle 2

Beeinflussung des Längenwachstums von Soja "Maple Arrow" bei Nachauflauf-Blattbehandlung

| Beispiel Nr. | mg a.S./Gefäß | relative Wuchshöhen* in % |
|---|---|---|
| CCC | 1,5 | 93,9 |
| 110 | 1,5 | 85,7 |

* bezogen auf unbehandelte Pflanzen

Tabelle 3

Beeinflussung des Längenwachstums von Sommergerste "Aramio" bei Vorauflauf-Bodenbehandlung

| Beispiel Nr. | mg a.S./Gefäß | relative Wuchshöhen* in % |
|---|---|---|
| CCC | 6 | 83,4 |
| 189 | 6 | 77,0 |

* bezogen auf unbehandelte Pflanzen

Tabelle 4

Beeinflussung des Längenwachstums von Sonnenblumen "Spanners Allzweck" bei Nachauflauf-Blattbehandlung

| Beispiel Nr. | mg a.S./Gefäß | relative Wuchshöhen* in % |
|---|---|---|
| CCC | 6 | 83,1 |
| 189 | 6 | 77,8 |

* bezogen auf unbehandelte Pflanzen

Tabelle 5

Beeinflussung des Längenwachstums von Sommerraps "Petranova" bei Nachauflauf-Blattbehandlung

| Beispiel Nr. | mg a.S./Gefäß | relative Wuchshöhen* in % |
|---|---|---|
| CCC | 6 | 92,2 |
| 181 | 6 | 75,4 |
| 182 | 6 | 79,0 |
| 188 | 6 | 79,2 |
| 189 | 6 | 75,3 |

* bezogen auf unbehandelte Pflanzen

Tabelle 6

Beeinflussung des Längenwachstums von Soja "Labrador" bei Nachauflauf-Blattbehandlung

| Beispiel Nr. | mg a.S./Gefäß | relative Wuchshöhen* in % |
|---|---|---|
| CCC | 0,5 | 98,3 |
| | 1,5 | 92,2 |
| 189 | 0,5 | 86,2 |
| | 1,5 | 83,7 |

* bezogen auf unbehandelte Pflanzen

Die in den Gewächshausversuchen für die in den Tabellen wiedergegebenen Tests verwendeten Pflanzen setzten sich aus folgenden Arten zusammen:

Weizen (Triticum aestivum), Gerste (Hordeum vulgare), Reis (Oryza sativa), Sonnenblume (Helanthus

annus), Raps (Brassia napus) und Soja (Glycine max.).

## Patentansprüche

1. Verwendung von Azolylmethyloxiranen der allgemeinen Formel I

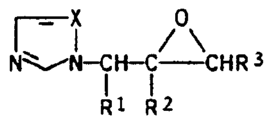

I

in der die Substituenten folgenden Bedeutung haben:

$R^1$ Wasserstoff oder ein Halogenatom,

$R^2$, $R^3$ $C_1$-$C_8$-Alkyl, welches einen der folgenden Reste tragen kann: Dioxanyl oder Phenyl, wobei der Phenylrest seinerseits ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy und/oder $C_1$-$C_4$-Halogenalkoxy,

$C_3$-$C_8$-Cycloalkyl, $C_5$-$C_8$-Cycloalkenyl, Tetrahydropyranyl oder Norbornyl, wobei diese cyclischen Reste ein bis drei der folgenden Substituenten tragen können: $C_1$-$C_4$-Alkyl und/oder Halogen,

Phenyl, Biphenyl, Naphthyl oder Pyridyl, wobei diese aromatischen Reste ein bis fünf Halogenatome und/oder ein bis drei der folgenden Substituenten tragen können: $C_1$-$C_4$-Alkyl, $C_1$-$C_4$-Halogenalkyl, $C_1$-$C_4$-Alkoxy, $C_1$-$C_4$-Halogenalkoxy, Phenoxy, Phenylsulfonyl, Amino, $C_1$-$C_4$-Alkylamino, Di-$C_1$-$C_4$-Alkylamino und/oder Nitro,

X Stickstoff oder eine Methingruppe CH

oder deren landwirtschaftlich brauchbaren Salze als Herbizide.

2. Verwendung von Azolylmethyloxiranen der allgemeinen Formel I gemäß Anspruch 1, in denen $R^1$ nicht Wasserstoff bedeutet, als Pflanzenwachstumsregulator.

3. Verfahren zur Bekämpfung unerwünschten Pflanzenwachstums, dadurch gekennzeichnet, daß man eine herbizid wirksame Menge eines Azolylmethyloxirans I gemäß Anspruch 1 auf die Samen, die Pflanzen und/oder deren Lebensraum einwirken läßt.

4. Verfahren zur Regulierung des Pflanzenwachstums, dadurch gekennzeichnet, daß man eine regulativ wirksame Menge eines Azolylmethyloxirans der Formel I, in der $R^1$ nicht Wasserstoff bedeutet, auf die Samen, die Pflanzen und/oder deren Lebensraum einwirken läßt.

## Claims

1. Use of an azolylmethyloxirane of the formula I

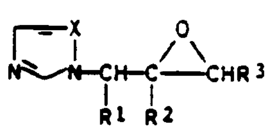

I,

where $R^1$ is hydrogen or halogen, $R^2$ and $R^3$ are each $C_1$-$C_8$-alkyl which may bear one of the following radicals: dioxanyl or phenyl, and the phenyl radical in turn may bear from one to five halogen atoms and/or one to three of the following groups: $C_1$-$C_4$-alkyl, $C_1$-$C_4$-haloalkyl, $C_1$-$C_4$-alkoxy and/or $C_1$-$C_4$-haloalkoxy; $R^2$ and $R^3$ are further $C_3$-$C_8$-cycloalkyl, $C_5$-$C_8$-cycloalkenyl, tetrahydropyranyl or norbornyl, where these cyclic radicals may bear from one to three of the following substituents: $C_1$-$C_4$-alkyl and/or halogen, $R^2$ and $R^3$ are further phenyl, biphenyl, naphthyl or pyridyl, where these aromatic radicals may bear from one to five halogen atoms and/or from one to three of the following substituents: $C_1$-$C_4$-alkyl, $C_1$-$C_4$-haloalkyl, $C_1$-$C_4$-alkoxy, $C_1$-$C_4$-haloalkoxy, phenoxy, phenylsulfonyl,amino, $C_1$-$C_4$-alkylamino, di-$C_1$-$C_4$-alkylamino and/or nitro and X is nitrogen or a methine group CH, or of an agriculturally useful salt thereof, as a herbicide.

2. Use of an azolylmethyloxirane of the formula I as claimed in claim 1 where $R^1$ is not hydrogen, as a plant growth regulator.

3. A process for controlling undesirable plant growth, wherein a herbicidally effective amount of an azolylmethyloxirane I as claimed in claim 1 is allowed to act on the seed, plants and/or their habitat.

4. A process for regulating plant growth, wherein a regulatory amount of an azolylmethyloxirane of the formula I where $R^1$ is not hydrogen is allowed to act on the seed, plants and/or their habitat.

## Revendications

1. Utilisation d'azolylméthyloxyranne de formule générale I

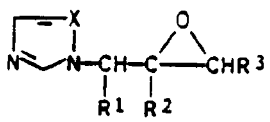

I

dans laquelle les symboles ont les significations suivantes :

$R^1$ représente l'hydrogène ou un atome d'halogène,

$R^2$, $R^3$ représentent chacun un groupe alkyle en C1-C8 qui peut porter l'un des substituants suivants : dioxannyle ou phényle, ce dernier pouvant lui-même porter de 1 à 5 atomes d'halogènes et/ou de 1 à 3 des substituants suivants : les groupes alkyle en C1-C4, halogénoalkyle en C1-C4, alcoxy en C1-C4 et/ou halogénoalcoxy en C1-C4,

un groupe cycloalkyle en C3-C8, cycloalcényle en C5-C8, tétrahydropyrannyle ou norbornyle, ces groupes cycliques pouvant porter de 1 à 3 des substituants suivants : les groupes alkyle en C1-C4 et/ou les halogènes,

un groupe phényle, biphényle, naphtyle ou pyridyle, ces groupes aromatiques pouvant porter de 1 à 5 atomes d'halogènes et/Ou de 1 à 3 des substituants suivants : les groupes alkyle en C1-C4, halogénoalkyle en C1-C4, alcoxy en C1-C4, halogénoalcoxy en C1-C4, phénoxy, phénylsulfonyle, amino, alkylamino en C1-C4, di-(alkyle en C1-C4)-amino et/ou nitro,

X représente l'azote ou un groupe méthine CH

ou de leurs sels acceptables pour les usages agricoles en tant qu'herbicides.

2. Utilisation des azolylméthyloxirannes de formule générale I de la revendication 1 dans laquelle $R^1$ a une signification autre que l'hydrogène, en tant que régulateurs de la croissance des végétaux.

3. Procédé pour combattre les croissances de végétaux indésirables, caractérisé en ce que l'on fait agir une quantité herbicide efficace d'un azolylméthyloxiranne I de la revendication 1 sur les semences, les plantes et/Ou leur habitat.

4. Procédé pour la régulation de la croissance des végétaux, caractérisé en ce que l'on fait réagir une quantité régulatrice efficace d'un azolylméthyloxiranne de formule I dans laquelle $R^1$ a une signification autre que l'hydrogène sur les semences, les plantes et/ou leur habitat.